# EUROPEAN PATENT APPLICATION

(11) **EP 3 011 889 A1**
(43) Date of publication of application: **27.04.2016**
(21) Application number: 13887582.8
(22) Date of filing: 27.12.2013
(51) Int. Cl.: A61B 1/00, G02B 23/24

(54) **ENDOSCOPE, OVERTUBE, AND ENDOSCOPE SYSTEM**

(30) Priority: 20.06.2013 JP 2013129609
(71) Applicant: Olympus Corporation, Shibuya-ku Tokyo 151-0072 (JP)
(72) Inventor: OKAZAKI, Yoshiro, Tokyo 151-0072 (JP); HIBINO, Hiroki, Tokyo 151-0072 (JP)
(74) Representative: Schicker, Silvia
(86) International application number: PCT/JP2013/085090
(87) International publication number: WO 2014/203432

(57) **Abstract**

Provided is an endoscope (1) including an inserted portion (2) that has, sequentially from a distal-end side, a distal-end portion (6), a bending portion (7), and a flexible portion (8); a wire (5) that has an exposed portion (5a) that is disposed outside the distal-end portion of the flexible portion (8) along the longitudinal direction and whose positions, at a first position and a second position that is separated from the first position on the base-end side, are restrained with respect to the distal-end portion of the flexible portion (8) in a radial direction; and a wire adjusting portion (12) that changes the length between the first position and the second position of the wire (5).

## Description

### {Technical Field}

The present invention relates to an endoscope, an overtube, and an endoscope system.

### {Background Art}

In the related art, there are known endoscopes provided with, between rigid distal-end portions that accommodate optical systems for capturing an image of a subject and elongated flexible portions having flexibility, bending portions that change the orientation of the distal-end portions (for example, see Patent Literature 1).

On the other hand, in endoscopic heart surgery, there is a known method in which the heart is treated in the pericardial cavity by percutaneously inserting an endoscope into the pericardial cavity (for example, see Patent Literature 2).

### {Citation List}

### {Patent Literature}

{PTL 1} Japanese Unexamined Patent Application, Publication No. 2011-19792
{PTL 2} U.S. Patent Application, Publication No. 2004/0064138, Specification

### {Summary of Invention}

### {Technical Problem}

However, the pericardial cavity is a thin space that is flanked by the heart and the pericardium covering the heart and that spreads along the heart surface and has a unique shape that is completely different from those of other body cavities (for example, digestive tract, thoracic cavity, and abdominal cavity). Inside the pericardial cavity having such a shape, the degree-of-freedom of movement of the inserted portion is restricted. In particular, because the inserted portion that is inserted into the pericardial cavity by passing through the pericardium prevents lateral movement at the portion at which the pericardium is passed through, the operation of the inserted portion is limited to movement forward, backward, and in the circumferential direction and to bending of the bending portion. As a result, there is a problem in that the movable range of the distal-end portion is restricted, and thus, there is a region that is difficult to reach with the distal-end portion.

The present invention has been conceived in light of the above-described circumstances, and an object thereof is to provide an endoscope, an overtube, and an endoscope system with which it is possible to enhance the degree-of-freedom of movement of the distal-end portion of an inserted portion and to increase the movable range of the distal-end portion.

### {Solution to Problem}

In order to achieve the above-described object, the present invention provides the following solutions.

A first aspect of the present invention is an endoscope including an inserted portion that has, sequentially from a distal-end side, a distal-end portion, a bending portion that changes an orientation of the distal-end portion, and an elongated flexible portion having flexibility; a wire that has an exposed portion that is disposed outside the distal-end portion of the flexible portion along a longitudinal direction of the flexible portion, and in which positions of the exposed portion, at a predetermined first position and a predetermined second position that is separated from the first position on a base-end side, are restrained with respect to the distal-end portion of the flexible portion in a radial direction; and a wire adjusting portion that changes a length between the first position and the second position of the wire.

With the first aspect of the present invention, when the length between the first position and the second position of the exposed portion of the wire is changed by the wire adjusting portion, a tensile force is generated in the wire between the first position and the second position where the position of the wire is restrained with respect to the flexible portion in the radial direction of the flexible portion, and, as a result, the distal-end portion of the flexible portion is bent so that the first position is brought close to the second position. In this way, by controlling the bent shape of not only the bending portion but also that of the distal-end portion of the flexible portion, it is possible to enhance the degree-of-freedom of movement of the distal-end portion and to increase the movable range of the distal-end portion.

Note that, with the present invention, a state in which the wire is restrained with respect to the flexible portion between the first position and the second position in the radial direction of the flexible portion refers to a state in which the movement of the wire in the circumferential direction of the flexible portion may be possible but the movement thereof in the radial direction of the flexible portion is practically not possible.

The above-described first aspect may be provided with a second-position adjusting portion that changes the second position in the longitudinal direction of the flexible portion.

By doing so, it is possible to change the orientation of the distal-end portion by changing the second position.

In the above-described first aspect, the flexible portion may be provided with an outer cylinder into which the flexible portion is inserted so as to be movable in the longitudinal direction; and a draw-out port that is formed at a distal end of the outer cylinder and from which a distal-end portion of the wire passing through the interior of the outer cylinder is drawn out, wherein the second-position adjusting portion moves the outer cylinder in the longitudinal direction.

By doing so, it is possible to change the position of the distal end of the outer cylinder, which corresponds to the second position, by means of a simple manipulation that merely moves the outer cylinder in the longitudinal direction.

In the above-described first aspect, the outer cylinder may have an inner diameter that allows a gap into which the wire can be inserted to be formed between an inner circumferential surface thereof and an outer circumferential surface of the flexible portion, and the wire may pass through the gap and may be drawn out from an opening of the gap provided at the distal end of the outer cylinder. Alternatively, in the above-described first aspect, the outer cylinder may have a first pathway, into which the flexible portion is inserted, and a second pathway, into which the wire is inserted, that are formed so as to pass therethrough in the longitudinal direction.

By doing so, it is possible to simplify the structure of the outer cylinder.

The above-described first aspect may be provided with a tube that accommodates the wire in a movable manner in the longitudinal direction, wherein the flexible portion may have a groove that is formed in the longitudinal direction at an outer surface thereof, and that accommodates the tube so as to be movable in the longitudinal direction, and the second-position adjusting portion may move the tube in the groove in the longitudinal direction with respect to the wire.

By doing so, it is possible to change the position of the distal end of the tube, which corresponds to the second position, by means of a simple manipulation that merely moves the tube in the longitudinal direction. In addition, because the wire is accommodated inside the flexible portion, it is possible to reduce the diameter as compared with a configuration that includes the outer cylinder.

The above-described first aspect may be provided with a plurality of the wires, and the plurality of the wires may be restrained with respect to the flexible portion at the common first position, and may be restrained with respect to the flexible portion at the second positions that differ from each other in the longitudinal direction.

By doing so, by selecting a wire whose length is to be changed by means of the wire adjusting portion among the plurality of wires, it is possible to change the movable range and the movable direction of the distal-end portion when the flexible portion is bent.

The above-described first aspect may be provided with a first-position adjusting portion that changes the first position in the longitudinal direction of the flexible portion.

By doing so, the position in the longitudinal direction of a portion of the flexible portion to be bent is changed by the first-position adjusting portion. Accordingly, it is possible to laterally change the movable range of the distal-end portion with respect to the straight portion of the flexible portion when the flexible portion is bent.

The above-described first aspect may be provided with a circumferential-direction-position adjusting portion that changes relative positions of the first position and second position in a circumferential direction of the flexible portion.

By doing so, it is possible to prevent the wire from winding around the outer circumferential surface of the flexible portion by adjusting the relative positions of the first position and the second position so that the first position and the second position are positioned at the same side in the circumferential direction.

A second aspect of the present invention is an overtube including a cylindrical main unit that has a pathway that is formed so as to pass therethrough in a longitudinal direction and into which a wire and an elongated flexible portion provided in an endoscope are inserted; and a securing portion that is provided at a base-end portion of the main unit and that secures an intermediate position of the wire to the main unit.

With the second aspect of the present invention, the overtube is attached to the flexible portion, the distal end of the wire that is inserted into the pathway of the main unit and drawn out from the distal end of the main unit is attached to the flexible portion, the base-end portion of the wire, which is drawn out from the base end of the main unit, is secured by the securing portion in the pulled state, and thus, it is possible to control bending of the flexible portion. Accordingly, it is possible to enhance the degree-of-freedom of movement of the distal-end portion of the inserted portion and to increase the movable range of the distal-end portion.

In the above-described second aspect, the main unit may be provided with an inner tube into which the flexible portion is inserted and an outer tube into which the inner tube is inserted in a movable manner in a longitudinal direction, wherein the size of the outer tube in the longitudinal direction may be smaller than the size of the inner tube in the longitudinal direction.

By doing so, by moving the outer tube in the longitudinal direction with respect to the inner tube, it is possible to change the movable range of the distal-end portion in the longitudinal direction of the flexible portion when the flexible portion is bent.

In the above-described second aspect, the main unit may have a groove that is formed at an outer surface in a longitudinal direction.

By doing so, the wire is inserted into the tube, the tube is inserted into the groove of the main unit, the tube is moved in the groove in the longitudinal direction with respect to the wire, and thus, it is possible to change the movable range of the distal-end portion in the longitudinal direction of the flexible portion when the flexible portion is bent.

A third aspect of the present invention is an endoscope system including an endoscope that has, sequentially from a distal-end side, a distal-end portion, a bending portion that changes an orientation of the distal-end portion, and an elongated flexible portion having flexibility; an overtube according to any one of the above, in which the flexible portion of the endoscope is inserted into the pathway of the main unit; a wire that is inserted into the pathway of the overtube, that is drawn out from the opening of the pathway provided at the distal end of the main unit, and whose distal end is connected to the flexible portion at the distal end of the flexible portion or in the vicinity thereof; and a wire adjusting portion that changes a length between the distal end of the wire and the opening of the pathway.

### {Advantageous Effects of Invention}

The present invention affords an advantage in that it is possible to enhance the degree-of-freedom of movement of a distal-end portion of an inserted portion and to increase the movable range of the distal-end portion.

### {Brief Description of Drawings}

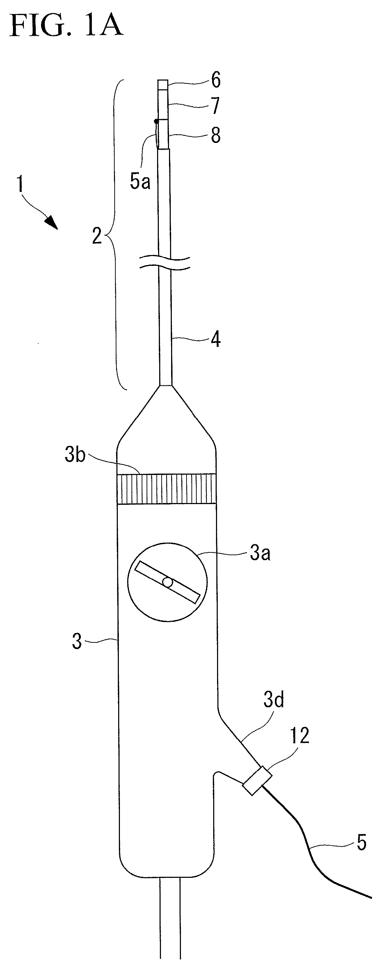

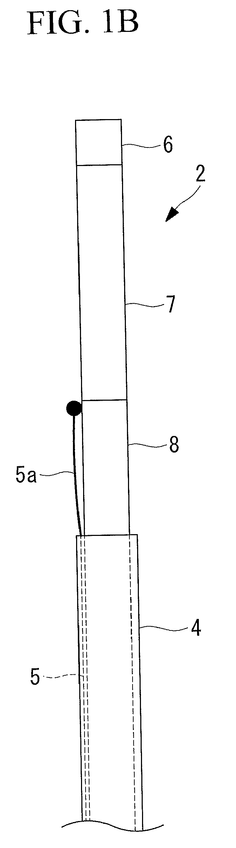

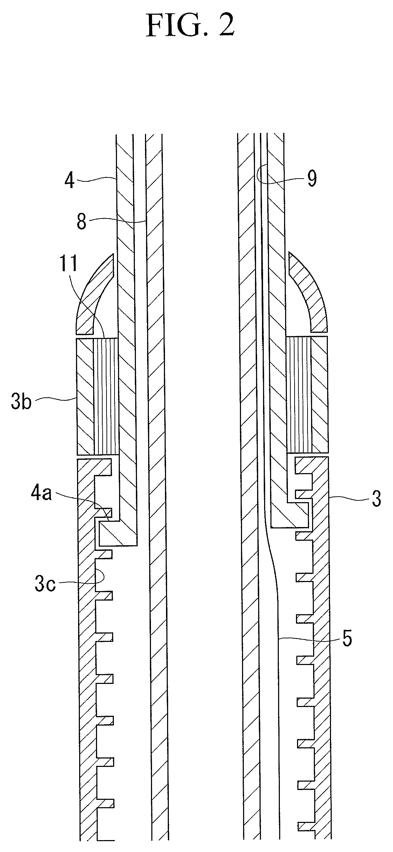

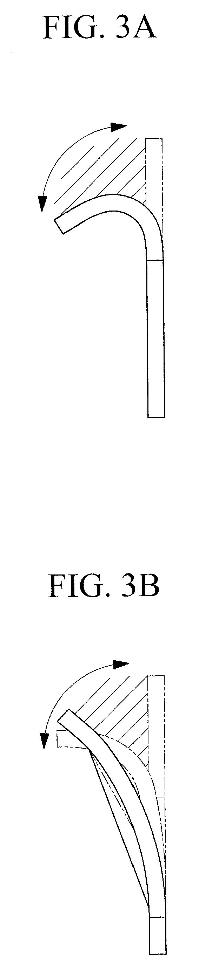

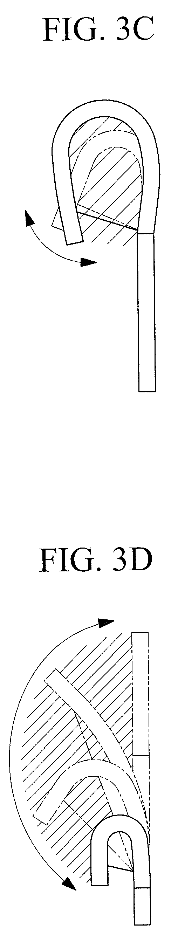

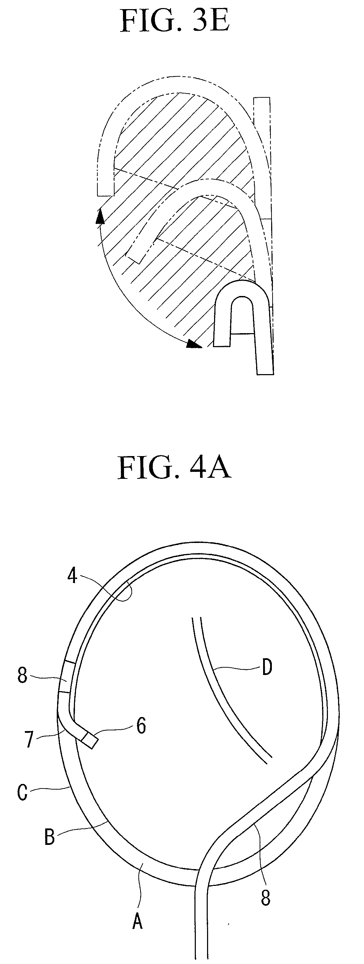

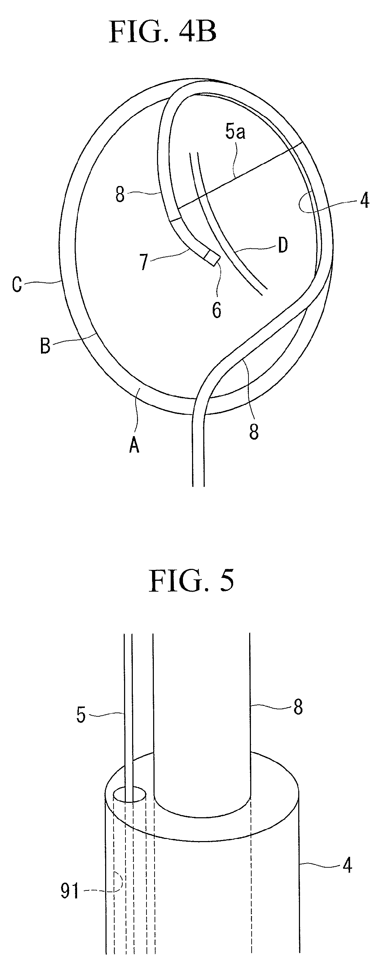

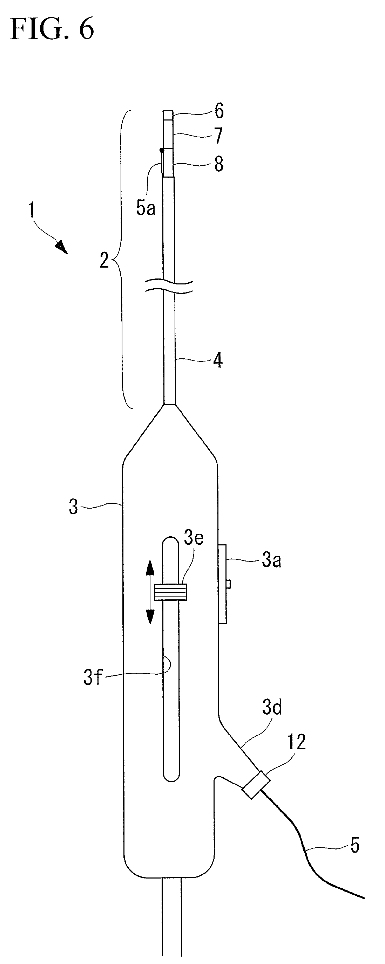

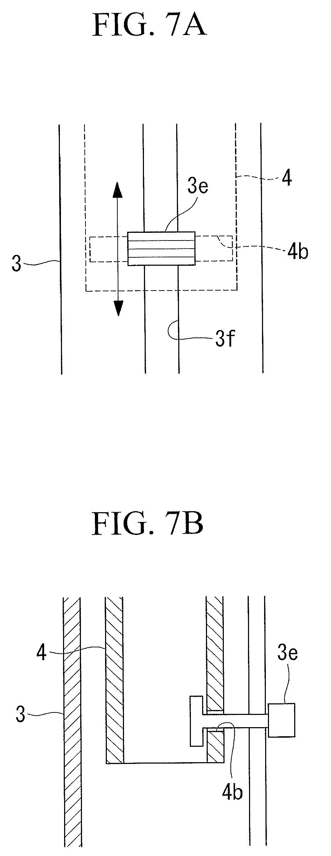

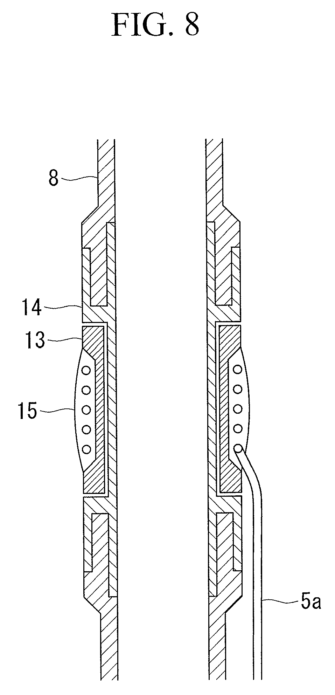

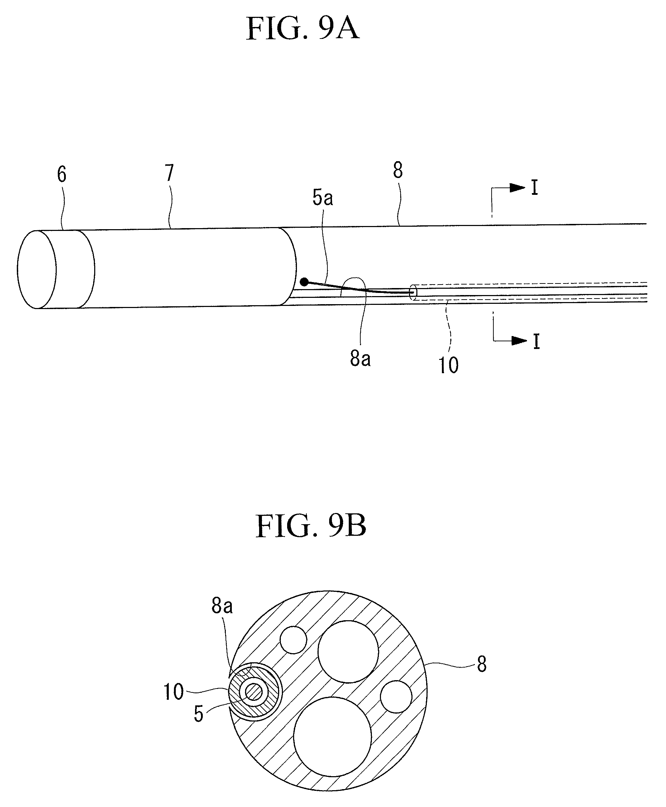

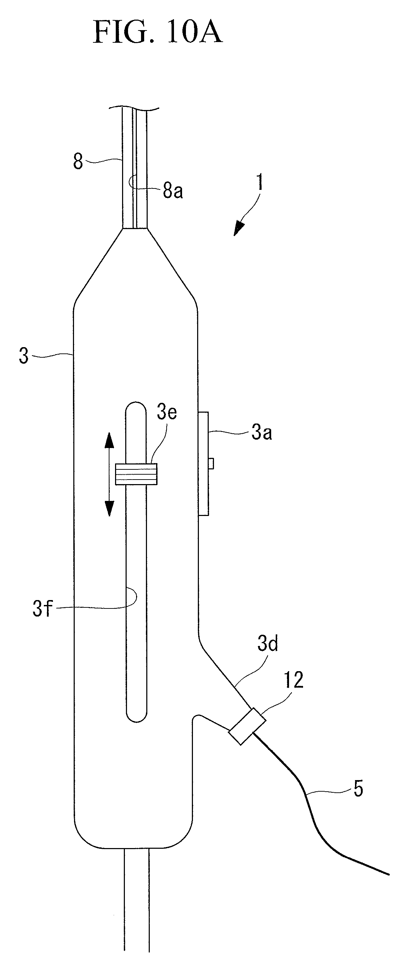

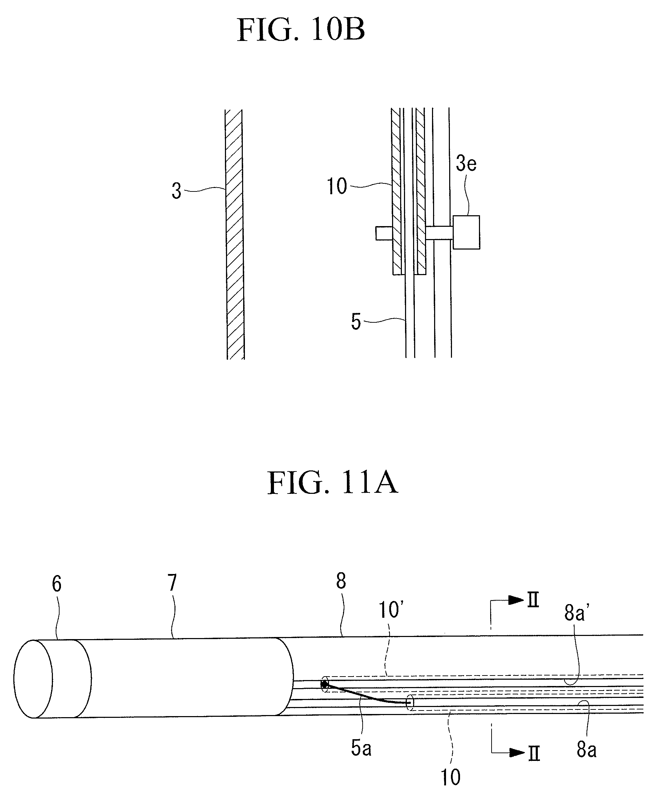

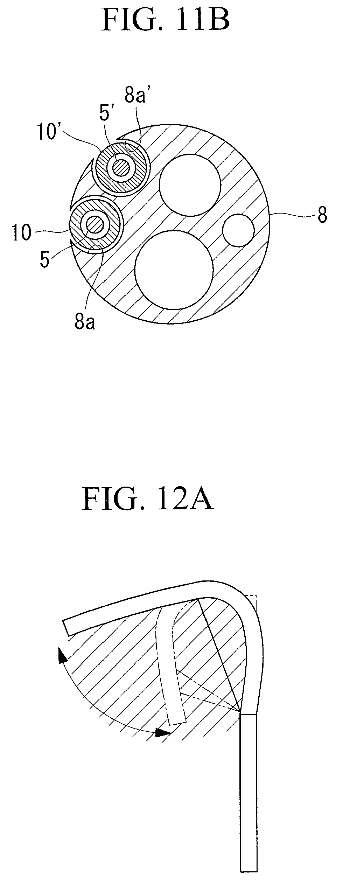

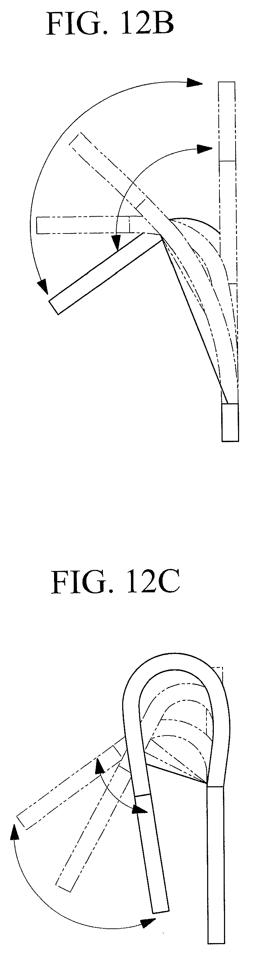

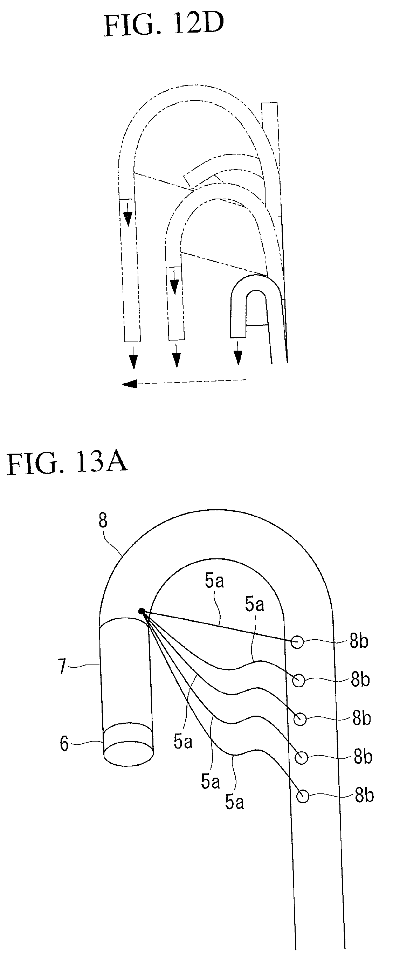

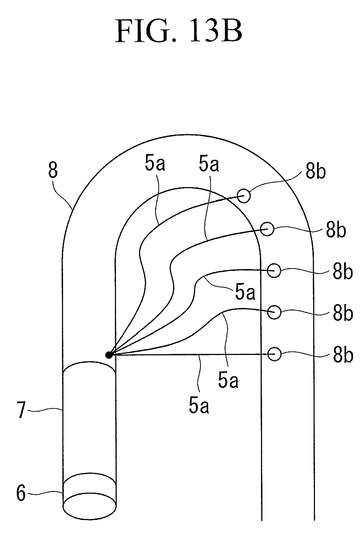

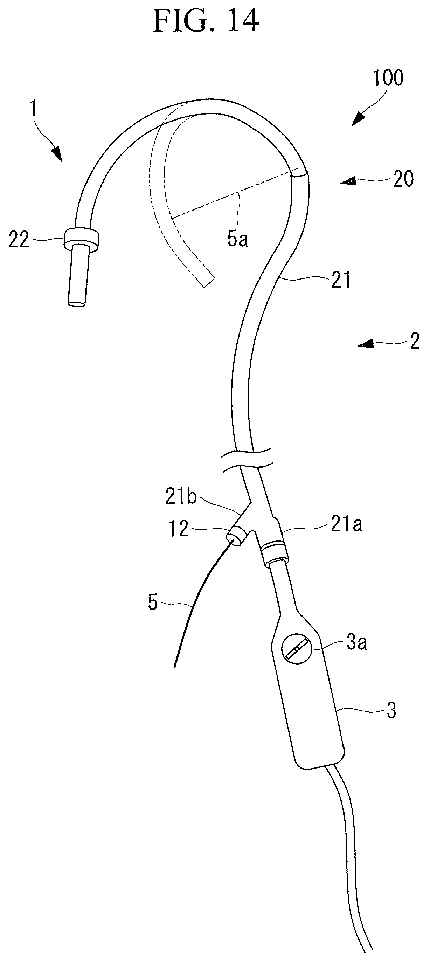

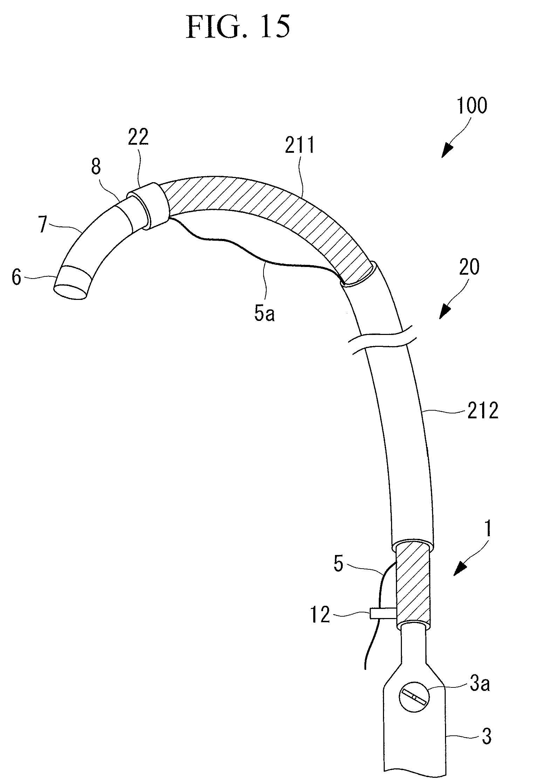

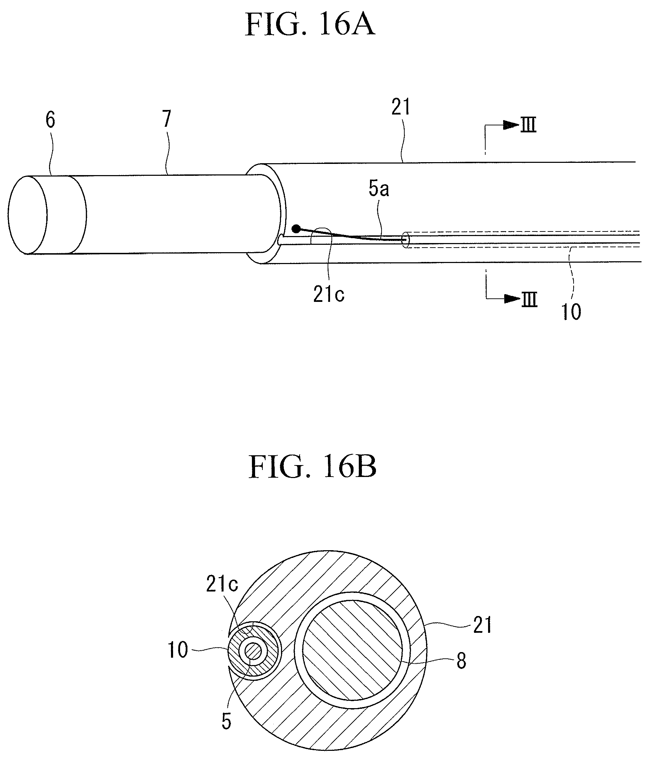

{Fig. 1A} Fig. 1A is an overall configuration diagram of an endoscope according to a first embodiment of the present invention.
{Fig. 1B} Fig. 1B is an enlarged view of a distal-end portion of the endoscope according to the first embodiment of the present invention.
{Fig. 2} Fig. 2 is a partial longitudinal sectional view showing the internal configuration of the endoscope in Fig. 1.
{Fig. 3A} Fig. 3A is a diagram for explaining a movable range and a movable direction of the distal-end portion of the endoscope in Fig. 1.
{Fig. 3B} Fig. 3B is a diagram for explaining the movable range and the movable direction of the distal-end portion of the endoscope in Fig. 1.
{Fig. 3C} Fig. 3C is a diagram for explaining the movable range and the movable direction of the distal-end portion of the endoscope in Fig. 1.
{Fig. 3D} Fig. 3D is a diagram for explaining the movable range and the movable direction of the distal-end portion of the endoscope in Fig. 1.
{Fig. 3E} Fig. 3E is a diagram for explaining the movable range and the movable direction of the distal-end portion of the endoscope in Fig. 1.
{Fig. 4A} Fig. 4A is a diagram for explaining the method of using the endoscope in Fig. 1, showing a state in which a flexible portion thereof is placed in a U-shape.
{Fig. 4B} Fig. 4B is a diagram for explaining a method of using the endoscope in Fig. 1, showing a state in which the distal-end portion is moved sideward by further bending the flexible portion.
{Fig. 5} Fig. 5 is a diagram showing a modification of wire placement.
{Fig. 6} Fig. 6 is an overall configuration diagram showing a modification of the endoscope in Fig. 1.
{Fig. 7A} Fig. 7A is a side view showing the configuration of a circumferential-direction adjusting portion provided in the endoscope in Fig. 5.
{Fig. 7B} Fig. 7B is a lateral sectional view showing the configuration of the circumferential-direction adjusting portion provided in the endoscope in Fig. 5.
{Fig. 8} Fig. 8 is a longitudinal sectional view showing another configuration of the circumferential-direction adjusting portion provided in the endoscope in Fig. 5.
{Fig. 9A} Fig. 9A is a configuration diagram of a distal-end portion of an endoscope according to a second embodiment of the present invention.
{Fig. 9B} Fig. 9B is a lateral sectional view of the distal-end portion of the endoscope in Fig. 9A, taken along I-I.
{Fig. 10A} Fig. 10A is a side view showing the configuration of a second-position adjusting portion provided in the endoscope in Fig. 9.
{Fig. 10B} Fig. 10B is a lateral sectional view of the second-position adjusting portion in Fig. 10A.
{Fig. 11A} Fig. 11A is a configuration diagram of a distal-end portion of an endoscope according to a third embodiment of the present invention.
{Fig. 11B} Fig. 11B is a lateral sectional view of the distal-end portion of the endoscope in Fig. 11A, taken along II-II.
{Fig. 12A} Fig. 12A is a diagram for explaining a movable range and a movable direction of the distal-end portion of the endoscope in Fig. 11.
{Fig. 12B} Fig. 12B is a diagram for explaining the movable range and the movable direction of the distal-end portion of the endoscope in Fig. 11.
{Fig. 12C} Fig. 12C is a diagram for explaining the movable range and the movable direction of the distal-end portion of the endoscope in Fig. 11.
{Fig. 12D} Fig. 12D is a diagram for explaining the movable range and the movable direction of the distal-end portion of the endoscope in Fig. 11.
{Fig. 13A} Fig. 13A is a configuration diagram of a distal-end portion of an endoscope according to a fourth embodiment of the present invention.
{Fig. 13B} Fig. 13B is configuration diagram of the distal-end portion of the endoscope according to the fourth embodiment of the present invention.
{Fig. 14} Fig. 14 is an overall configuration diagram of an endoscope system according to a fifth embodiment of the present invention.
{Fig. 15} Fig. 15 is an overall configuration diagram of a modification of the endoscope system in Fig. 14.
{Fig. 16A} Fig. 16A is a configuration diagram of a distal-end portion of another modification of the endoscope system in Fig. 14.
{Fig. 16B} Fig. 16B is a lateral sectional view of the distal-end portion of the endoscope system in Fig. 16A, taken along III-III.

### {Description of Embodiments}

### {First Embodiment}

An endoscope 1 according to a first embodiment of the present invention will be described below with reference to Figs. 1A to 8.

As shown in Fig. 1A, the endoscope 1 according to this embodiment is provided with an elongated inserted portion 2 that is inserted into a body interior, a manipulation portion 3 that is provided at the base-end side of the inserted portion 2, and an outer cylinder 4 and a wire 5 that are attached to the inserted portion 2.

The inserted portion 2 is provided with, sequentially from the distal-end side, a short, rigid distal-end portion 6, a bending portion 7 that changes the orientation of the distal-end portion 6, and an elongated flexible portion 8 that has flexibility, which allows the flexible portion 8 to bend in accordance with the shape of tissue inside the body.

The distal-end portion 6 is provided with, in the interior thereof, an optical system (not shown) for capturing an image of a subject, and the endoscope 1 is configured so as to acquire an endoscope image of the subject by using this optical system.

The flexible portion 8 normally takes a substantially straight shape, but is elastically bendable in accordance with an external force.

The manipulation portion 3 is provided with an angle lever 3a that controls the bending operation of the bending portion 7. When a user operates the angle lever 3a, the bending portion 7 is deformed from the straight shape to a bent shape, and, in addition, the bending angle thereof is also changed.

The outer cylinder 4 is a tubular member having flexibility, and the flexible portion 8 is inserted into the interior of the outer cylinder 4. The outer cylinder 4 has a larger inner diameter than the outer diameter of the flexible portion 8 so as to ensure a large enough gap, into which the wire 5 can be inserted, between an inner circumferential surface of the outer cylinder 4 and an outer circumferential surface of the flexible portion 8, and, also, so that the outer cylinder 4 can be moved in the longitudinal direction with respect to the flexible portion 8. In addition, the outer cylinder 4 is formed of an X-ray opaque material, for example, a plastic or a metal, thus making it visible in an X-ray image.

Furthermore, the outer cylinder 4 is configured so as to be moved in the longitudinal direction with respect to the flexible portion 8 by means of a dial-style outer-cylinder manipulation portion (second-position adjusting portion) 3b provided in the manipulation portion 3. The outer-cylinder manipulation portion 3b is provided in the main unit of the manipulation portion 3 so as to be rotatable in the circumferential direction. In addition, as shown in Fig. 2, the outer-cylinder manipulation portion 3b is coupled with the outer cylinder 4 via gears 11 so that the outer cylinder 4 can be moved in the longitudinal direction while transmitting the rotational motion to the outer cylinder 4. At the base end of the outer cylinder 4, a thread 4a that protrudes radially outward is provided, and a spiraling spiral groove 3c into which the thread 4a is inserted is formed inside the manipulation portion 3. By doing so, when the user rotates the outer-cylinder manipulation portion 3b, the thread 4a is moved along the spiral groove 3c so as to cause the outer cylinder 4 to move forward or backward in the longitudinal direction while rotating.

The wire 5 is inserted into the gap 9, which is ensured to have a sufficient size between the outer circumferential surface of the flexible portion 8 and the inner circumferential surface of the outer cylinder 4, along the longitudinal direction of the flexible portion 8. The gap 9 may be filled with a lubricant (not shown, but, for example, physiological saline, hyaluronic acid gel, prostaglandin solution or the like) so that the wire 5 is moved smoothly in the gap 9.

The distal-end portion of the wire 5 is drawn out from a draw-out port, which is an opening of the gap 9 provided at the distal end of the outer cylinder 4, and is disposed outside the flexible portion 8, and the distal end of the wire 5 is secured to the flexible portion 8 at the distal end of the flexible portion 8 or in the vicinity thereof (first position). The base-end portion of the wire 5 is drawn out from a wire port 3d provided in the manipulation portion 3 and is disposed outside the manipulation portion 3. The user can bend the distal-end portion of the flexible portion 8 by pulling the base-end portion of the wire 5.

At this time, because the base end of an exposed portion 5a of the wire 5, which is disposed outside the distal-end portion of the flexible portion 8, is restrained in terms of its position in the radial direction with respect to the flexible portion 8 at the draw-out port of the outer cylinder 4, a tensile force toward the base end of the exposed portion 5a acts on the distal end of the wire 5 that is being pulled. As a result, the flexible portion 8 is bent so that the distal-end portion 6 points toward the base end of the exposed portion 5a (that is, the distal end of the outer cylinder 4).

The wire port 3d is provided with a securing portion (wire adjusting portion) 12 for securing an intermediate position of the wire 5 to the manipulation portion 3. By securing the wire 5 to the manipulation portion 3 by means of the securing portion 12, the amount by which the wire 5 is pulled can be kept constant. The securing portion 12 is provided with, for example, a cylindrical rubber piece into which the wire 5 is inserted, and, by squashing this rubber piece in a radial direction, the wire 5 is immobilized by means of a frictional force.

Here, by adjusting the position (second position) of the distal end of the outer cylinder 4 by means of the outer-cylinder manipulation portion 3b as well as by adjusting the length of the exposed portion 5a of the wire 5 by pulling the wire 5, as shown in Figs. 3B to 3E, it is possible to control the bent shape of the distal-end portion of the flexible portion 8, thus making it possible to change the position and the orientation of the distal-end portion 6. Fig. 3A shows the movable range of the distal-end portion 6 when only the bending portion 7 is bent. Figs. 3A to 3E schematically show the shapes of the flexible portion 8.

As shown in Fig. 3B, when the outer cylinder 4 is retracted to the base-end side thereof in a state in which the length of the exposed portion 5a is held constant, it is possible to change the orientation of the distal-end portion 6, that is, the viewing-field direction, to substantially 90° by bending the flexible portion 8 to a relatively small bending angle. On the other hand, as shown in Fig. 3C, when the distal-end portion of the flexible portion 8 is pushed out from inside the outer cylinder 4 in the state in which the length of the exposed portion 5a is held constant, it is possible to change the orientation of the distal-end portion 6 to substantially 180° by bending the distal-end portion of the flexible portion 8 to an even greater bending angle. On the other hand, as shown in Fig. 3D, when the outer cylinder 4 is retracted and the wire 5 is also pulled, it is possible to arbitrarily change the radius of curvature of the flexible portion 8. Furthermore, by applying a motion that pushes out the distal-end portion of the flexible portion 8 from inside the outer cylinder 4 in Fig. 3D, it is possible to bend the flexible portion 8 with a greater radius of curvature, as shown in Fig. 3E.

Next, the operation of the thus-configured endoscope 1 will be described by using a case in which a heart B is treated inside a pericardial cavity A as an example.

To treat the heart B by using the endoscope 1 according to this embodiment, first, the user inserts the endoscope 1 into the body from below the xiphoid process, makes the distal-end portion 6 pass through a pericardium C near the apex cordis, and places the inserted portion 2 in the pericardial cavity A in a U-shape, as shown in Fig. 4A. At this time, it is preferable that the outer cylinder 4 be disposed at a position at which the wire 5 is satisfactorily hidden so that the wire 5 does not hinder the operation of the inserted portion 2.

When in the U-shape shown in Fig. 4A, due to a restoring force of the inserted portion 2 tending to return to the straight shape, the inserted portion 2 is pressed against the inner surface of the pericardium C. Specifically, because a force that spreads the inserted portion 2 in the radial direction is generated in the U-shaped inserted portion 2, the distal-end portion 6 and the flexible portion 8 are subjected to a biasing force that separates them from each other.

By performing manipulations shown in Figs. 3B to 3E depending on the target position in the heart B, the user can move the distal-end portion 6 to a desired position by resisting the biasing force that acts on the distal-end portion 6.

In particular, in the case of observing or treating a region surrounded by the U-shaped inserted portion 2, for example, a coronary artery D in Fig. 4A, the user laterally and substantially horizontally moves the distal-end portion 6 to the coronary artery D according to the following procedures.

First, the outer cylinder 4 is retracted by rotating the outer-cylinder manipulation portion 3b to place the distal end of the outer cylinder 4 at a position that is substantially opposite the distal-end portion 6 with the coronary artery D interposed therebetween. Next, the base-end portion of the wire 5, which is disposed outside the manipulation portion 3, is pulled. By doing so, the distal end of the flexible portion 8 is pulled by the wire 5 toward the distal end of the outer cylinder 4, which further bends the distal-end portion of the flexible portion 8, and thus, the distal-end portion 6 is laterally moved toward the coronary artery D by resisting the biasing force, as shown in Fig. 4B. The distance by which the distal-end portion 6 is moved and the position to which the distal-end portion 6 is moved at this time can be checked by using at least one of an X-ray image and an endoscope image. After moving the distal-end portion 6 to the vicinity of the coronary artery D, the position of the wire 5 is secured by using the securing portion 12 provided in the wire port 3d. By doing so, the bent shape of the flexible portion 8 is maintained.

In the case in which other regions of the heart B need to be observed or treated, the positions of the outer cylinder 4 and the wire 5 are readjusted. Specifically, when the wire 5 is released by using the securing portion 12, the distal-end portion 6 is moved in the direction away from the flexible portion 8 in accordance with the restoring force of the flexible portion 8, and thus, the inserted portion 2 returns to the state shown in Fig. 4A. Subsequently, the above-described procedures are repeated.

As has been described above, with this embodiment, by adjusting the position of the distal end of the outer cylinder 4, from which the wire 5 is drawn out, and the length of the exposed portion 5a of the wire 5, the bent shape of the distal-end portion of the flexible portion 8 is controlled, and, by doing so, it is possible to enhance the degree-of-freedom of movement of the distal-end portion 6, and it is also possible to increase the movable range of the distal-end portion 6. As a result, there is an advantage in that it is possible to easily place the distal-end portion 6 even in regions that are difficult to reach with the distal-end portion 6 solely by means of operation of the inserted portion 2 in the front-to-back direction and the circumferential direction and bending operation of the bending portion 7, and thus, it is possible to increase the area that can be observed/treated. In particular, because it is also possible to laterally and substantially horizontally move the distal-end portion 6, it is possible to easily place the distal-end portion 6 even in a region surrounded by the U-shaped inserted portion 2, which has been extremely difficult to reach with a conventional distal-end portion 6.

Note that, although the wire 5 is inserted into the gap 9 between the outer cylinder 4 and the flexible portion 8 in this embodiment, alternatively, as shown in Fig. 5, a hole (second pathway) 91 may be formed in a side wall of the outer cylinder 4 so as to pass therethrough from the distal end to the wire port 3d, and the wire 5 may be inserted into the hole 91. In this configuration, it is preferable that, so that it is possible to recognize the circumferential-direction position of the opening (draw-out port) of the hole 91 at the distal end of the outer cylinder 4, a marker indicating the circumferential-direction position of the opening be provided at the base end of the outer cylinder 4.

In addition, this embodiment may be provided with a circumferential-direction-position adjusting portion that prevents the exposed portion 5a from winding around the outer circumferential surface of the flexible portion 8 by adjusting the relative positions between the distal end and the base end of the exposed portion 5a of the wire 5 in the circumferential direction.

When the flexible portion 8 is twisted inside the body, the positional relationship in the circumferential direction is changed between the distal end and the base end of the exposed portion 5a. When the wire 5 is pulled in a state in which the distal end and the base end of the exposed portion 5a are not positioned on the same side in the circumferential direction, that is, in a state in which the wire 5 is wound around the outer circumferential surface of the flexible portion 8, an undesirable force acts on the flexible portion 8, which may make it impossible to manipulate the flexible portion 8 as intended. Therefore, by positioning the distal end and the base end of the exposed portion 5a at the same side in the circumferential direction by means of the circumferential-direction-position adjusting portion, it is possible to maintain the bending operability of the manipulation portion 3.

Fig. 6 shows an endoscope provided with a circumferential-direction-position adjusting portion that rotates the base end of the exposed portion 5a in the circumferential direction. In this configuration, the outer cylinder 4 is provided so as to be rotatable in the circumferential direction with respect to the flexible portion 8, and the manipulation portion 3 is provided with a knob (second-position adjusting portion, circumferential-direction-position adjusting portion) 3e that moves the outer cylinder 4 in the circumferential direction and the front-to-back direction, instead of the dial-style outer-cylinder manipulation portion 3b.

As shown in Figs. 7A and 7B, the knob 3e is inserted, in a movable manner, into the interior of a slit 4b, which is formed in the circumferential direction over half the circumference of the outer cylinder 4, and the interior of a slit 3f, which is formed in the manipulation portion 3 along the longitudinal direction of the flexible portion 8, and a portion thereof is disposed outside the manipulation portion 3 via the slit 3f of the manipulation portion 3. By doing so, the user can move the opening in a 180° range in the circumferential direction by rotationally operating the base-end portion of the outer cylinder 4 in the circumferential direction, and he/she can move the outer cylinder 4 forward or backward by operating the knob 3e in the longitudinal direction.

Fig. 8 shows the configuration of the circumferential-direction-position adjusting portion that rotates the distal end of the exposed portion 5a in the circumferential direction. This circumferential-direction-position adjusting portion is provided with a ring-like rotating member 13 that is attached at the distal-end portion of the flexible portion 8 so as to be freely rotatable in the circumferential direction of the flexible portion 8. The rotating member 13 is supported in a freely rotatable manner by a rotation receiving portion 14 attached to the outer circumferential surface of the flexible portion 8. The distal end of the wire 5 is secured to the rotating member 13 by means of an adhesive 15. When the flexible portion 8 is bent in the state in which the distal end and the base end of the exposed portion 5a are not positioned at the same side in the circumferential direction, winding of the wire 5 on the flexible portion 8 is undone by rotating the rotating member 13 so as to move the distal end to the same side as the base end.

### {Second Embodiment}

Next, an endoscope according to a second embodiment of the present invention will be described with reference to Figs. 9A to 10B. Note that points that differ from those of the above-described first embodiment will mainly be described for this embodiment, and configurations in common with those of the first embodiment will be given the same reference signs and descriptions thereof will be omitted.

As shown in Figs. 9A and 9B, the endoscope according to this embodiment mainly differs from that of the first embodiment in that a tube (second-position adjusting portion) 10 that accommodates the wire 5 in the longitudinal direction in a movable manner is provided as a mechanism for adjusting the position of the base end of the exposed portion 5a of the wire 5, instead of the outer cylinder 4.

In Fig. 9B, holes other than a groove 8a indicate channels in which a treatment tool, wiring, a light guide, and so forth are placed. Although the groove 8a whose lateral cross-section has a substantially circular shape as shown in Fig. 9B, it is possible to appropriately change the lateral cross-sectional shape of the groove 8a. For example, the lateral cross-sectional shape of the groove 8a may be a T-shape, a polygon, or an elliptical shape.

The flexible portion 8 has the groove 8a that is formed in the outer surface and that extends in the longitudinal direction, and the tube 10 is accommodated in the groove 8a so as to be movable in the longitudinal direction. By moving the tube 10 in the groove 8a in the longitudinal direction, the position of the base end of the exposed portion 5a, that is, the position of the opening (draw-out port) at the distal end of the tube 10 from which the wire 5 is drawn out is changed.

Figs. 10A and 10B show a mechanism (second-position adjusting portion) for moving the tube 10 in the longitudinal direction. Although this mechanism has a similar configuration to that of the mechanism shown in Figs. 6 to 7B, the former differs from the latter in that a shaft 31e of the knob 3e is secured to the base-end portion of the tube 10. By moving the knob 3e along the slit 3f, the user can move the tube 10 in the longitudinal direction with respect to the wire 5 and the flexible portion 8.

With the thus-configured endoscope 1 according to this embodiment, in addition to the advantages afforded by the first embodiment, there is an advantage in that, by accommodating the wire 5 inside the flexible portion 8, it is possible to reduce the diameter of the flexible portion 8 as compared with that of the endoscope 1 according to the first embodiment. In addition, because the position of the base end of the exposed portion 5a in the circumferential direction is set by the groove 8a, a certain relative positions are always maintained between the distal end and the base end of the exposed portion 5a in the circumferential direction. By doing so, it is possible to prevent the wire 5 from winding around the outer circumferential surface of the flexible portion 8.

### {Third Embodiment}

Next, an endoscope 1 according to a third embodiment of the present invention will be described with reference to Figs. 11A to 12D. Note that points that differ from those of the above-described first and second embodiments will mainly be described for this embodiment, and common configurations with those of the first and second embodiments will be given the same reference signs and descriptions thereof will be omitted.

The endoscope 1 according to this embodiment is a modification of the endoscope 1 of the second embodiment, and, as shown in Figs. 11A and 11B, another set of a wire 5', a tube 10' and a groove 8a' is provided as the first-position adjusting portion that changes the position (first position) of the distal end of the exposed portion 5a in the longitudinal direction of the flexible portion 8.

Distal ends of the two wires 5 and 5' are connected to each other. The tube 10' accommodates substantially the entire length of the wire 5'. In addition, the tube 10' and the wire 5' are secured to each other so as to move as a single unit in another groove 8a' formed near the groove 8a.

With the thus-configured endoscope 1 according to this embodiment, in addition to the advantages afforded by the first and second embodiments, an advantage is afforded in that it is possible to further enhance the degree-of-freedom of movement of the distal-end portion 6 by changing the position of the distal end of the exposed portion 5a in the longitudinal direction of the flexible portion 8.

Specifically, as shown in Fig. 12A, by retracting the tube 10' and pushing out the wire 5, it is possible to observe a position laterally separated further from the flexible portion 8. On the other hand, as shown in Fig. 12B, by moving the tube 10 in a state in which the tube 10' is retracted, although the bending angle range of the distal-end portion of the flexible portion 8 is the same, the movable range of the distal-end portion 6 is increased as compared with Fig. 3B. On the other hand, as shown in Fig. 12C, by pushing out the flexible portion 8 in a state in which the tube 10' is retracted, although the bending angle range of the distal-end portion of the flexible portion 8 is the same, the movable range of the distal-end portion 6 is increased as compared with Fig. 3D. On the other hand, as shown in Fig. 12D, by moving both tubes 10 and 10, it is possible to laterally move the distal-end portion 6 with respect to the substantially straight portion of the flexible portion 8 while maintaining the distal-end portion 6 in a certain orientation. Note that Figs. 12A to 12D schematically show the shapes of the flexible portion 8.

### {Fourth Embodiment}

Next, an endoscope according to a fourth embodiment of the present invention will be described with reference to Figs. 13A and 13B. Note that points that differ from those of the above-described first to third embodiments will mainly be described for this embodiment, and configurations in common with those of the first to third embodiments will be given the same reference signs, and descriptions thereof will be omitted.

As shown in Figs. 13A and 13B, the endoscope according to this embodiment mainly differs from those of the first to third embodiments in that a plurality (five in the illustrated example) of wires 5 are provided.

Distal ends of the plurality of wires 5 are secured to the flexible portion 8 at the same position. The flexible portion 8 has a plurality of draw-out ports 8b that are provided in a side surface thereof so as to be arranged in a row in the longitudinal direction, and the exposed portions 5a of the plurality of wires 5 are individually drawn out from different draw-out ports 8b. The plurality of draw-out ports 8b communicate with a common cavity formed inside the flexible portion 8, and the plurality of wires 5 are disposed in the common cavity. Alternatively, a plurality of cavities may be formed inside the flexible portion 8, and the plurality of wires 5 may individually be disposed in different cavities.

The manipulation portion 3 is provided with a plurality of wire ports 3d, and the base-end portions of the individual wires 5 are individually drawn out to outside the manipulation portion 3 from different wire ports 3d. In this embodiment, when the flexible portion 8 is lightly bent, as shown in Fig. 13A, the wire 5 drawn out from the draw-out port 8b at the distal-end side is pulled, and, when the flexible portion 8 is strongly bent, as shown in Fig. 13B, the wire 5 drawn out from the draw-out port 8b at the base-end side is pulled, thus making it possible to more appropriately control the lateral and substantially horizontal movement of the distal-end portion 6.

With the thus-configured endoscope according to this embodiment, in addition to the advantages afforded by the first embodiment, there is an advantage in that it is possible to facilitate manufacturing by simplifying the structure as compared with those of the first and second embodiments.

In this embodiment, it is preferable that the plurality of wires 5 possess X-ray imaging properties that are different from each other. By doing so, it is possible to distinguish the plurality of wires 5 from each other in an X-ray image.

### {Fifth Embodiment}

Next, an overtube 20 according to a fifth embodiment of the present invention and an endoscope system 100 provided with the same will be described with reference to Figs. 14 to 16B. Although the endoscope 1 that is provided with the wire 5 and the outer cylinder 4 or the tube 10 has been described in the above-described first to third embodiments, it is also possible to increase the movable range of the distal-end portion 6 in the case in which the wire 5 and the outer cylinder 4 or the tube 10 are configured as a separate unit from the endoscope 1, as in this embodiment.

As shown in Fig. 14, the endoscope system 100 according to this embodiment is provided with the endoscope 1, and the overtube 20 to be placed on the inserted portion 2 provided in the endoscope 1. The overtube 20 is provided with a cylindrical main unit 21 and a distal-end adaptor 22 that are attached to the flexible portion 8 of the endoscope 1.

The endoscope 1 is a general soft endoscope.

As with the above-described outer cylinder 4, the main unit 21 has an inner diameter that allows a gap into which the wire 5 can be inserted to be formed between the inner circumferential surface thereof and the outer circumferential surface of the flexible portion 8. In addition, the main unit 21 is provided with, at the base-end side thereof, a flexible portion attaching portion 21a that is secured to the flexible portion 8 and a wire port 21b. The flexible portion attaching portion 21a attaches the main unit 21 to the flexible portion 8 by using the same mechanism as the securing portion 12 provided in the wire port 3d described above. The wire port 21b is configured in the same manner as the above-described wire port 3d and is provided with the securing portion 12 for securing the wire 5 to the main unit 21.

The distal-end adaptor 22 is formed of, for example, a ring-shaped member and is attached to the distal end of the flexible portion 8 or in the vicinity thereof. In addition, the distal-end adaptor 22 has a wire attaching portion (not shown), and the distal end of the wire 5 is attached to the wire attaching portion. Note that the wire 5 may simply be tied to the distal-end adaptor 22.

Next, the operation of the thus-configured overtube 20 and endoscope system 100 will be described.

To use the overtube 20 according to this embodiment, the overtube 20 is attached to the inserted portion 2 according to the following procedures. First, the wire 5 is inserted into the main unit 21, the distal end of the wire 5 is attached to the distal-end adaptor 22, and the base-end portion of the wire 5 is drawn out from the wire port 21b to be placed outside the main unit 21. Next, the inserted portion 2 is inserted into the interiors of the main unit 21 and the distal-end adaptor 22, the distal-end adaptor 22 is attached to the distal-end portion of the flexible portion 8, and the main unit 21 is attached to the flexible portion 8 at a position away from the distal-end adaptor 22 toward the base end.

Subsequently, the user can control bending of the flexible portion 8 by pulling the base-end portion of the wire 5 that is drawn out from the wire port 3d, as indicated by the one-dot-chain line in Fig. 14.

With the thus-configured overtube 20 and endoscope system 100 according to this embodiment, in addition to the advantage afforded by the above-described first embodiment, there is an advantage in that it is possible to use a general-purpose endoscope 1, and, in addition, it is possible to achieve simple, low-cost manufacturing.

Note that, although the main unit 21 is formed of a single member in this embodiment, alternatively, as shown in Fig. 15, the main unit 21 may have a two-layer structure provided with an inner tube 211 and an outer tube 212 into which the inner tube 211 is inserted.

The distal-end adaptor 22 is secured to the distal end of the inner tube 211. In addition, the inner tube 211 has a length that accommodates substantially the entire longitudinal size of the flexible portion 8.

The outer tube 212 is disposed outside the inner tube 211 so as to be movable in the longitudinal direction with respect to the inner tube 211, and has a shorter length than the inner tube 211.

A gap into which the wire 5 can be inserted is formed between the inner circumferential surface of the outer tube 212 and the outer circumferential surface of the inner tube 211, and the wire 5 extends to the base-end side of the inserted portion 2 by passing through the gap from the distal end of the outer tube 212 to the base end thereof. Alternatively, the outer tube 212 may have the same structure as does the outer cylinder 4 shown in Fig. 5. Specifically, the wire 5 may be inserted into a hole formed in the outer tube 212, instead of the gap between the outer tube 212 and the inner tube 211.

The user can bend the flexible portion 8 in an arbitrary U-shape by adjusting the position of the base end of the exposed portion 5a of the wire 5 by moving the outer tube 212 to the base-end side and by subsequently pulling the base-end portion of the wire 5.

In addition, in this embodiment, the main unit 21 may have the same structure as does the flexible portion 8 shown in Figs. 9A and 9B. Specifically, as shown in Figs. 16A and 16B, a groove 21c that is similar to the groove 8a may be formed in the outer surface of the main unit 21, and the tube 10 in which the wire 5 is accommodated may be inserted into the groove 21c.

By doing so, there is an advantage in that it is possible to reduce the diameter of the main unit 21, and, in addition, that it is possible to prevent the wire 5 from winding around the outer circumferential surface of the main unit 21.

### {Reference Signs List}

1 endoscope
2 inserted portion
3 manipulation portion
3a angle lever
3b outer-cylinder manipulation portion (second-position
adjusting portion)
3c spiral groove
3d wire port
3e knob (second-position adjusting portion, circumferential-direction-position adjusting portion)
3f slit
4 outer cylinder
4a thread
4b slit
5 wire
5a exposed portion
6 distal-end portion
7 bending portion
8 flexible portion
8a groove
9 gap
91 hole (second pathway)
10 tube
11 gear
12 securing portion (wire adjusting portion)
13 rotating member
14 rotation receiving portion
15 adhesive
20 overtube
21 main unit
21a flexible portion attaching portion
21b wire port
21c groove
211 inner tube
212 outer tube
22 distal-end adaptor
A pericardial cavity
B heart
C pericardium
D coronary artery

## Claims

1. An endoscope comprising:
an inserted portion that has, sequentially from a distal-end side, a distal-end portion, a bending portion that changes an orientation of the distal-end portion, and an elongated flexible portion having flexibility;
a wire that has an exposed portion that is disposed outside the distal-end portion of the flexible portion along a longitudinal direction of the flexible portion, and in which positions of the exposed portion, at a predetermined first position and a predetermined second position that is separated from the first position on a base-end side, are restrained with respect to the distal-end portion of the flexible portion in a radial direction; and
a wire adjusting portion that changes a length between the first position and the second position of the wire.

2. The endoscope according to Claim 1, further comprising:
a second-position adjusting portion that changes the second position in the longitudinal direction of the flexible portion.

3. The endoscope according to Claim 2, further comprising:
an outer cylinder into which the flexible portion is inserted so as to be movable in the longitudinal direction; and
a draw-out port that is formed at a distal end of the outer cylinder and from which a distal-end portion of the wire passing through the interior of the outer cylinder is drawn out,
wherein the second-position adjusting portion moves the outer cylinder in the longitudinal direction.

4. The endoscope according to Claim 3, wherein
the outer cylinder has an inner diameter that allows a gap into which the wire can be inserted to be formed between an inner circumferential surface thereof and an outer circumferential surface of the flexible portion, and
the wire passes through the gap, and is drawn out from an opening of the gap provided at the distal end of the outer cylinder.

5. The endoscope according to Claim 3, wherein
the outer cylinder has a first pathway, into which the flexible portion is inserted, and a second pathway, into which the wire is inserted, that are formed so as to pass therethrough in the longitudinal direction.

6. The endoscope according to Claim 2, further comprising:
a tube that accommodates the wire in a movable manner in the longitudinal direction,
wherein the flexible portion has a groove that is formed in the longitudinal direction at an outer surface thereof, and that accommodates the tube so as to be movable in the longitudinal direction, and
the second-position adjusting portion moves the tube in the groove in the longitudinal direction with respect to the wire.

7. The endoscope according to Claim 1 wherein
a plurality of the wires are provided, and
the plurality of the wires are restrained with respect to the flexible portion at the common first position, and are restrained with respect to the flexible portion at the second positions that differ from each other in the longitudinal direction.

8. The endoscope according to any one of Claims 1 to 7, further comprising:
a first-position adjusting portion that changes the first position in the longitudinal direction of the flexible portion.

9. The endoscope according to any one of Claims 1 to 8, further comprising:
a circumferential-direction-position adjusting portion that changes relative positions of the first position and second position in a circumferential direction of the flexible portion.

10. An overtube comprising:
a cylindrical main unit that has a pathway that is formed so as to pass therethrough in a longitudinal direction and into which a wire and an elongated flexible portion provided in an endoscope are inserted; and
a securing portion that is provided at a base-end portion of the main unit and that secures an intermediate position of the wire to the main unit.

11. The overtube according to Claim 10, wherein
the main unit is provided with an inner tube into which the flexible portion is inserted and an outer tube into which the inner tube is inserted in a movable manner in a longitudinal direction,
wherein the size of the outer tube in the longitudinal direction is smaller than the size of the inner tube in the longitudinal direction.

12. The overtube according to Claim 10, wherein the main unit has a groove that is formed at an outer surface in a longitudinal direction.

13. An endoscope system comprising:
an endoscope that has, sequentially from a distal-end side, a distal-end portion, a bending portion that changes an orientation of the distal-end portion, and an elongated flexible portion having flexibility;
the overtube according to any one of Claims 10 to 12, in which the flexible portion of the endoscope is inserted into the pathway of the main unit;
a wire that is inserted into the pathway of the overtube, that is drawn out from the opening of the pathway provided at the distal end of the main unit, and whose distal end is connected to the flexible portion at the distal end of the flexible portion or in the vicinity thereof; and
a wire adjusting portion that changes a length between the distal end of the wire and the opening of the pathway.
